(19) Europäisches Patentamt / European Patent Office / Office européen des brevets

(11) **EP 4 162 885 A1**

(12) **EUROPEAN PATENT APPLICATION**

(43) Date of publication:
**12.04.2023 Bulletin 2023/15**

(21) Application number: **21200851.0**

(22) Date of filing: **05.10.2021**

(51) International Patent Classification (IPC):
***A61B 17/135*** (2006.01)

(52) Cooperative Patent Classification (CPC):
**A61B 17/135; A61B 5/022;** A61B 2017/00707;
A61B 2090/064; A61B 2090/0809

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DAVIDOIU, Valentina-Geta**
  **Eindhoven (NL)**
• **SCHULZE, Andreas**
  **Eindhoven (NL)**
• **KUNT, Stanislav**
  **Eindhoven (NL)**

(74) Representative: **Philips Intellectual Property & Standards**
**High Tech Campus 52**
**5656 AG Eindhoven (NL)**

(54) **DEVICE, SYSTEM AND METHOD FOR DETECTING A DEFECT IN MATERIAL AND/OR WORKMANSHIP OF AN INFLATABLE CUFF**

(57)    The present invention relates to a device, system and method for detecting a defect in material and/or workmanship of an inflatable cuff. Furthermore, the present invention relates to a phantom arm device. The device for detecting a defect in material and/or workmanship of an inflatable cuff comprises a target pressure input configured to obtain a target pressure signal, the target pressure signal indicating a target pressure within the cuff; a sensor input configured to obtain a cuff pressure signal from a sensor configured to measure a pressure within the cuff, the cuff pressure signal indicating a pressure within the cuff; an output configured to output a warning signal; and a processing unit configured to analyze the cuff pressure signal and the target pressure signal, to detect the of the cuff based on said analysis, and to control the output to output the warning signal if the defect is detected.

Fig. 1

EP 4 162 885 A1

**Description**

FIELD OF THE INVENTION

The present invention relates to a device, system and method for detecting a defect in material and/or workmanship of an inflatable cuff. Furthermore, the present invention relates to a phantom arm device.

BACKGROUND OF THE INVENTION

Blood pressure (BP) is one of the most important vital signs measured during almost every medical examination. Arterial blood pressure can either be obtained invasively via an arterial catheter or non-invasively (NIBP). The invasive BP monitoring is usually indicated only in the case of high-risk patients or in complex surgical procedures. Thus, the most used and popular method to measure BP is the non-invasive method (NIBP). Two distinct automatic NIBP methods are available on the market: a conventional one, which is deflation-based NIBP, and a second one, which is inflation-based NIBP (iNIBP). The purpose of iNIBP is to substantially reduce the measurement time and patient discomfort with respect to the conventional, deflation-based NIBP. In fact, using iNIBP instead of NIBP should reduce the average measurement time from 45 to 20 seconds, while maintaining the same measurement accuracy.

In most cases, BP is monitored non-invasively by occluding the upper arm using an oscillometric blood pressure cuff. The BP readings for the automated oscillometry technology are obtained, for example, in a hospital environment at fixed time intervals that can vary from 1 minute to couple of hours. BP measurements are these days very popular also for home monitoring where the user can have a measurement whenever it suits him or her.

Inaccurate BP measurements in general may result in misdiagnosis and hence may lead to cardiovascular complications or clinical deterioration. In fact, overestimating true blood pressure by 5 mmHg would lead to inappropriate treatment with antihypertension medications in almost 30 million Americans (see e.g. J. Handler, "The Importance of Accurate Blood Pressure Measurement", The Permanente Journal, Volume 13, No. 3, pp. 51-54, 2009).

The accuracy of a BP measurement and measurements of other vital signs such as hemodynamic parameters (e.g. cardiac output or stroke volume) performed using an inflatable cuff, can be greatly affected by defects in material and/or workmanship of the cuff.

The accuracy of a BP measurement, for example, can be affected by a sudden drop of the cuff pressure caused by an artefact called "velcro crack" or "cuff crack". A "velcro crack" is usually produced by the velcro material of the cuff, in particular by the velcro material of a closing element of the cuff, e.g. by the hook-and-loop fastener of the cuff. A "cuff crack" may be caused by other parts of the cuff, in particular other tissue parts of the cuff. For example, a cuff crack may be caused by a cuff comprising several layers of (different) materials which are not firmly attached to each other but can, to some extent, move relative to each other, particularly during inflation or deflation of the cuff. This may be the case in cuffs having a dual bladder-design where an inner bladder is inserted into the outer shell of the cuff. During the inflation/deflation of the internal bladder, the bladder may slightly move within the shell. In some cases, friction between the bladder and shell surfaces may lead to a cuff crack (slip-stick phenomenon). Similarly, a cuff crack may be caused by the slip-stick phenomenon caused by overlapping parts of the closing element of the cuff, which move against each other during an inflation or deflation of the cuff. Furthermore, closing elements comprising buttons, particularly push buttons, may open during inflation and likewise distort a measurement result.

Both (velcro crack and cuff crack) artefacts can be understood as a mechanical crack, for example the sudden opening of hooks and loops during an inflation process of the blood pressure cuff. If this artefact is very severe it can be heard as a distinctive "ripping" sound. Moreover, the crack artefacts may be superimposed on the true pressure signal and therefore a wrong estimate of the final blood pressure values (systolic and/or diastolic) will be obtained. Hence, the accuracy of vital sign measurements provided by using an inflatable cuff may be affected by defects of the material of the cuff or poor workmanship of the materials, particularly tissue(s), used for the cuff.

SUMMARY OF THE INVENTION

It is an object of the present invention to provide a device, system and method for detecting a defect in material and/or workmanship of an inflatable cuff and thus to ensure that a cuff used for a vital sign measurement (for example a BP measurement) provides accurate measurement results.

In a first aspect of the present invention a device for detecting a defect in material and/or workmanship of an inflatable cuff is presented, the device comprising:

a target pressure input configured to obtain a target pressure signal, the target pressure signal indicating a target pressure within the cuff;
a sensor input configured to obtain a cuff pressure signal from a sensor configured to measure a pressure within the cuff, the cuff pressure signal indicating a pressure within the cuff;
an output configured to output a warning signal; and
a processing unit configured to analyze the cuff pressure signal and the target pressure signal, to detect the defect of the cuff based on said analysis, and to control the output to output the warning signal if the defect is detected.

In a further aspect of the present invention a phantom arm device for detecting a defect in material and/or workmanship of an inflatable cuff is presented, the phantom arm device comprising:

- an inner cylinder comprising a first material,
- a middle layer comprising a second material, the middle layer being wrapped around a shell surface of the inner cylinder; and
- an outer layer comprising a third material, the outer layer being wrapped around the middle layer,

> wherein a hardness degree of the first material is higher than the hardness degree of the second material, and
> wherein the hardness degree of the second material is higher than the hardness degree of the third material.

In a further aspect of the present invention a system for detecting a defect in material and/or workmanship of an inflatable cuff is presented, the system comprising:

> a sensor configured to measure a pressure within the cuff wrapped around a rigid cylindrical form or a phantom arm device;
> a pressure-delivery unit configured provide a target pressure within the cuff; and
> a device for detecting a defect in material and/or workmanship of an inflatable cuff.

In yet further aspects of the present invention, there are provided a corresponding method and a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that defects in material and/or workmanship of the cuff, e.g. of the closing element of a cuff, such as a sudden opening of a hook-and-loop fastener, affect the pressure within a cuff. For example, if a cuff gets looser on an arm of a patient due to a sudden opening of the closing element the volume inside the cuff that can be inflated may become bigger and thus the pressure within the cuff may decrease suddenly at the time of such opening. Similarly, a sudden movement of the bladder within the shell of the cuff, caused by the slip-stick phenomenon, for example, may cause sudden changes in the volume of the inflatable part of the cuff and thus cause sudden changes in the pressure within the cuff.

Hence, by analyzing the pressure within the cuff, particularly with respect to a nominal pressure, i.e. the target pressure, deficiencies in the cuff material, such as the velcro material of the closing element, may be detected.

The proposed system, devices and method are very general and can be used for all existing commercial cuffs, but also for new prototypes to assess in an accurate, robust and reproducible way the quality of a cuff. In particular, material and design of a cuff can be analyzed with respect to their suitability for providing a reliable vital sign measurement and studies with humans to assess quality of a cuff can be avoided. The proposed method, device and system may also provide insights for development of new, improved cuffs. With the present invention new kinds of closing elements can be tested, for example new materials for hook-and-loop fasteners. Furthermore, deficiencies in the closing elements or deficiencies of shell and/or bladder material can be detected which cannot be observed using the naked eye. Apart from that, other features of a cuff, such as the design of the cuff can be assessed.

Therefore, the present invention allows for detecting cuffs suffering from malfunctions of their closing element or from defects of their material and for developing cuffs free of malfunctions and/or defects. Accordingly, the accuracy of vital sign measurements using a cuff can be improved. Since malfunctions and/or defects caused by the material or poor workmanship regarding the material of the cuff, such as velcro cracks, generally do not follow a particular pattern it is quite hard to be correct artefacts in a cuff pressure signal caused by these malfunction/defects by a software algorithm. However, the present invention may offer a systematic method to test cuffs for such malfunctions/defects and may thus help gain enough information to improve software algorithms for correcting cuff pressure signals.

In the device for detecting a defect in material and/or workmanship of an inflatable cuff the target pressure input may obtain the target pressure signal from a pressure-delivery unit, for example. The pressure-delivery unit may comprise an air-pump system configured to pump air into the interior of the cuff and thus to inflate the cuff. The target-pressure signal indicating a target pressure within the cuff may comprise information about the maximum (minimum) pressure the cuff is configured to be inflated to (deflated to) and a target pressure rate, i.e. inflation rate indicating the pressure at the which the cuff is inflated (deflated). From this information the processing unit may infer the target pressure (i.e. a nominal pressure that should be within the cuff at a particular point in time (during inflation or deflation)). The target pressure signal may likewise indicate a gas flow entering the cuff during inflation (or leaving the cuff during deflation) and the processing unit of the device may be configured to calculate a target pressure (nominal pressure) based on said signal and to analyze the cuff pressure signal based on the target pressure. However, the target pressure signal may already indicate the target pressure, i.e. the pressure that should be within the cuff at a certain point in time, without the need for the processing unit to perform further calculations.

In general, the target pressure signal may be regarded as a reference pressure signal for detecting defects. The target pressure signal may be derived from the device using information from a pressure delivery unit. Additionally, information from the measured cuff pressure signal may be used. However, there is also the possibility that the device infers or calculates the target pressure signal (solely) from the measured cuff pressure signal, using particular algorithms, for example. In this case, the target pressure input may be configured to obtain the target pressure signal by processing the cuff pressure signal, for example.

Apart from the target pressure signal the processing unit uses the cuff pressure signal recorded during the assessment (measurement) for defect detection. Usually, the cuff gets inflated at a given pressure rate to a predefined pressure and the cuff pressure signal is recorded during the inflation. The cuff pressure signal is then analyzed with respect to irregularities indicating defects of the cuff.

During a measurement time, particularly during inflation, deflation or during a time of constant pressure within the cuff, the sensor input may obtain the cuff pressure signal from a sensor configured to measure the pressure within the cuff. In general, the (pressure) sensor can be located anywhere as long as it is pneumatically connected to the cuff. For example, the sensor may be located (anywhere) in a pneumatic system connected to the cuff, e.g. a pneumatic system of the pressure delivery unit. However, other locations are likewise conceivable. For example, the sensor may be connected to a hose (tube) leading the air from a pump outlet to an outer connector of the cuff. In fact, a pressure sensor located directly within the cuff may result in the least pump (background) noise and the best sensitivity. However, such positioning may be difficult to realize since it would be necessary to modify the cuff to insert the pressure sensor (in the most cases).

A practicable location for good sensitivity may be directly at an inlet (for pressure measurement) of the cuff. In general, the location of the pressure sensor may depend on the technical feasibility and the desired sensitivity.

Both the cuff pressure signal and the target pressure signal may be analyzed by the processing unit of the device. For example, the processing unit may be configured to compare the cuff pressure signal with the target pressure signal, particularly during the measurement time, and to detect the defect based on said comparison. However, the processing unit may likewise check whether the pressure rate at which the cuff is inflated, as indicated by the target pressure signal, is constant and analyze the cuff pressure signal in view of said information. If it is analyzed that the cuff pressure signal does not represent a constant cuff pressure rate the defect may be detected.

In case a defect is detected, the output of the device is configured to provide a warning signal, particularly to an output interface, which may then issue the defect detected by the device.

In an embodiment, the device further comprises a control unit configured to control a pressure-delivery system to provide the target pressure within the cuff, wherein the control unit is configured to control the pressure-delivery unit to inflate the cuff at a predefined inflation rate and/or to deflate the cuff at a predefined deflation rate and/or to hold the target pressure at a predefined (constant) level.

The inflation rate and/or the deflation rate may be a constant rate. However, control unit may be configured to control the pressure-delivery unit to inflate and/or deflate the cuff at a variable rate. In particular, the control unit may be configured to control the pressure-delivery unit to inflate and/or deflate the cuff at a rate depending on a size of the cuff or a material of the cuff, for example. Furthermore, the rate may depend on a size of a rigid cylindrical form or a phantom arm device the cuff is wrapped around during the measurement. Preferably, the inflation rate ranges between 0.5 mmHg per second and 30 mmHg per second. Furthermore, the control unit may be configured to control the pressure-delivery unit to inflate and/or deflate the cuff and/or to hold a predefined pressure within the cuff for a predefined time.

In an embodiment of the device, the processing unit is configured to detect the defect based on a change in the cuff pressure signal, particularly a magnitude and/or shape of the change, more particularly a magnitude and/or shape of a drop or increase in the cuff pressure signal.

Preferably, the processing unit is configured to (calculate and) analyze a derivative of the cuff pressure signal and to detect the defect based on the derivative. For example, the processing unit may be configured to detect a negative impulse (part) in the derivative of the cuff pressure signal and to detect the defect if said impulse is larger than pump noise oscillations of the pressure-delivery unit, i.e. if said impulse is higher than a predetermined threshold, wherein the threshold may depend on the size of background noise oscillations. This way defects caused by the closing element or other parts of the cuff may not be confused with background noise.

In another embodiment the processing unit is configured to detect the defect if the change in the cuff pressure signal is higher than a threshold, wherein the threshold is a static threshold or an adaptable threshold. The processing unit may be configured to adapt the threshold with respect to background noise (caused by the pressure-delivery unit and the like), for example. In general, the (static or adaptable) threshold may be as low as 0.1 mmHg per second. Other thresholds, for example in the range between 0.1 mmHg and 5 mmHg or between 1 mmHg and 10 mmHg are conceivable as well. The threshold may be adapted depending on a sensitivity of a sensor used for measuring the cuff pressure signal and/or on a smallest defect of interest. By increasing the threshold, smaller cracks may be ignored but also false positive detections due to noise of the setup may decrease.

Preferably, the processing unit is configured to detect the defect based on a comparison of changes in the cuff pressure signal and the target pressure signal. In particular, the processing unit may be configured to detect the defect if a difference of changes the cuff pressure signal and the target pressure signal (at a particular time during the measurement time) is higher than a predetermined threshold. By comparing both signals detection of a malfunction (defect) caused by a pressure-delivery unit can be excluded. This is due to the fact that if the cuff pressure signal and the target pressure signal comprise a sudden change at the same time, the change in the cuff pressure signal may not be caused by a defect of the material and/or workmanship of the material, but by the change of the target pressure signal.

In an embodiment the processing unit is configured to detect the defect based on any of a spectral analysis of the cuff pressure signal, a matching of the cuff pressure signal with a template and an evaluation of a variability of a filtered cuff pressure signal.

In other words, the processing unit may be configured to perform a spectral analysis of the cuff pressure signal and to detect the defect based on said analysis. To be more specific, the cuff pressure signal may be analyzed with a (short-time) Fourier transformation or a similar spectral method. An increased amplitude on frequencies, typically indicative of a defect of the material or workmanship of the cuff, are then denoted as the defect.

Furthermore, the device may be configured to store or obtain various templates of previous cuff pressure signals, the templates comprising information about artefacts in these signals associated with defects of a cuff. To obtain such templates predefined (defect) patterns, i.e. short signal excerpts of a cuff pressure signal indicating a defect, are searched within an existing cuff pressure signal. The cuff pressure signals may be filtered beforehand. To search the excerpt a cross-correlation or other methods can be used. Subsequently, by comparing a cuff pressure signal with a template the processing unit may detect parallels and thus may detect the defect.

However, the processing unit of the device may further comprise a filter unit configured to filter the cuff pressure signal, wherein the filter unit comprises any of a band-pass filter, a low-pass filter and a high-pass filter, wherein the processing unit is configured to detect the defect based on an evaluation of a variability of the filtered signal. In particular, the processing unit may be configured to detect the defect based on a change in the filtered cuff pressure signal, more particularly based on a comparison of changes of the filtered cuff pressure signal and the target pressure signal.

In general, the pressure signal may be pre-processed and/or filtered before analyzing it. For example, high-frequent noise and pump-induced disturbances may be removed from the signals using a low-pass or band-stop filter. The cuff pressure signal may likewise be filtered by a high-pass filter to remove the inflation ramp resulting in a signal with removed trend (detrended cuff pressure signal). In this case changes in the signal are accentuated.

In an idealized setup with an ideal cuff without any defect, the resulting cuff pressure signal would be a horizontal line (a constant 0). Due to background noise, however, there are irregular oscillations around the baseline (0). A defect may be distinguished from such an oscillation if its amplitude is higher than the amplitude of the background noise. To get a score describing how much the cuff is affected by defect, an RMS (root mean square), peak-to-peak or similar computation of signal variability may performed for the detrended signal.

To be more precise, after detrending the cuff pressure signal may enter an algorithm which is configured to compare changes in the detrended pressure signal with a threshold that is adapting itself to the background noise in the signal at the same time. Whenever the threshold is exceeded, the processing unit detects (and records) a defect. Further information related to the defect may be recorded as well, such as its amplitude, duration, and/or pressure level where it occurred. On the other hand, several non-parametric features may computed from the detrended cuff pressure signal, evaluating the variability-unrest in the signal, e.g. root mean square (RMS), peak-to-peak (difference between the maximum and minimum in the signal). The more defects the higher the variability in the signal. An advantage of this method is that there is no need for any parameters and that is works with all kinds of defects that affect the detrended cuff pressure signal. However, defects may neither be localized nor analyzed with respect to particular characteristics.

In another embodiment the processing unit is configured to analyze the cuff pressure signal during any of inflation of the cuff, deflation of the cuff or an inflated state of the cuff.

Particularly during inflation of the cuff parts of the closing element can be ripped apart and cause false measurement results of vital signs such as BP.

In an embodiment the cuff is configured to be wrapped around a cylindrical form (and closed by the closing element, particularly during the measurement time), the cylindrical form comprising a rigid cylinder or a phantom arm device.

The cylindrical form is configured to imitate an arm of a human being. The cylindrical form, particularly the rigid cylinder, may comprise plastic material or metal. By wrapping the cuff around said (lifeless) form, distortions in the cuff pressure signal caused by a person's movements, pulse or any other kind of human factor can be avoided.

Although a simple rigid cylinder from any material that does not compress during an inflation can be used in assessing the quality of a cuff a phantom arm device comprising a soft outer layer has the advantage that a cuff wrapped around it (tightly) can expand towards the cylinder (contrary to a rigid cylinder which does not yield during inflation). Furthermore, the cuffs behavior during inflation or deflation is closer to the behavior of the cuff when wrapped around a human arm.

In another embodiment the defect comprises an opening of a closing element, particularly a sudden opening, wherein the closing element comprises any of two surfaces touching each other, a hook and loop fastener, a magnetic closure, and a button.

An example of a button may particularly comprise a push button. Accordingly, the defect may comprise a button coming off or hook coming loose from a loop. The defect may likewise comprise the slip-stick phenomenon, i.e. is the spontaneous jerking motion that can occur while two objects (surfaces) are sliding over each other. Any of these defects may cause a (sudden) change of the volume inside the cuff and thus a sudden change in the measured cuff pressure (and the cuff pressure signal).

In yet another embodiment the processing unit is configured to analyze the cuff pressure signal at different inflation rates and/or at different deflation rates. In particular, the control unit may be configured to control a pressure-delivery unit to inflate the cuff at an inflation rate of 6 mmHg/s, 15 mmHg/s and 25 mmHg/s and the processing unit may be configured to analyze the cuff pressure signal at (any of) these rates. This way the behavior of the cuff can be tested in more detail.

In still another embodiment the processing unit is configured to count a number of the defects detected, particularly during the measurement time. Furthermore, the processing unit may be configured to determine a total sum of the magnitude of all changes corresponding to a defect detected. Apart from that the processing unit may be configured to evaluate a level of the cuff pressure at which a defect is detected.

These analyses may help provide meaningful statistics with respect to the quality of tested cuffs. In particular, determining the total sum of the magnitude of all malfunctions may help assess the quality for a cuff. For example, if the total magnitude is very high, this is an indicator that the cuff suffers from severe defects and cannot be used to measure a vital sign in a reliable manner.

In an embodiment of the phantom arm device the first material comprises (rigid) polytetrafluoroethylene and the second and/or third material comprises silicone rubber, wherein a diameter of the inner cylinder is between 1cm and 3 cm, preferably 2 cm, and/or wherein a thickness of the middle layer is between 2 cm and 4 cm, preferably 3 cm, and/or wherein a thickness of the outer layer is between 0.5 mm and 1.5 mm, preferably 1 cm. This way, the geometry of a real human arm is mimicked in a precise manner. Preferably, the thickness of the middle layer and the outer layer is constant and the phantom arm device comprises a cylindrical shape.

In another embodiment the phantom arm device comprises one or more further layers. Any of these layers may be located below the middle layer or above the middle layer and or below the outer layer and or above the outer layer. A further layer may be wrapped around the whole inner cylinder or only around parts of the inner cylinder. The material of the one or more further layers may comprise silicone rubber, for example. In particular, the middle layer 6 and/or the outer layer may comprise a (medical) silicone elastomer. More particularly, the middle layer may comprise Silpuran® 2420 and the outer layer may comprise Silpuran® 2400.

In a preferred embodiment, the phantom arm device comprises a cylinder that has a diameter which is constant along the entire length of the phantom arm device. In other words, any point on the shell surface of the cylinder of the phantom arm device has the same distance to the inner cylinder.

In an embodiment of the system, the system further comprises a rigid cylindrical form or a phantom arm device as claimed herein, wherein the rigid cylindrical form or the phantom arm device is used to mimic a human arm and the cuff is wrapped around the rigid cylindrical form or the phantom arm device during the measurement time.

In an embodiment of the system the pressure-delivery unit is configured to maintain a predefined constant (target) pressure rate and/or to maintain a predefined constant gas flow rate. In general, pressure-delivery unit is configured to ensure a controlled inflation and/or deflation of the cuff. For example, the pressure-delivery unit may comprise programmable system which is configured to maintain a predefined constant pressure rate (mmHg/s). The programmable system may comprise a hardware module featuring universal signal inputs and outputs, running a user-defined algorithm in real-time and a personal computer running controlling, visualizing and recording software. The programmable system may be used to control inflation and/or deflation, to record a pressure of inflation and/or deflation, to process signals and to execute a particular algorithm on signals.

On the other hand, the pressure-delivery unit may comprise a (thermal) mass flow controller configured to maintain a constant flow (ml/s or standard cube centimeter per second) of gas into the cuff (independent from an input pressure). Via a controller unit in the mass flow controller a flow value can be defined, which then can be regulated via a regulator unit (e.g. an electronic valve). Hence, the mass flow controller may guarantee a well-defined inflation behavior. The advantage of a mass flow controller is the absence of an air pump (it uses a compressed air inflow) and therefore the background noise on the cuff pressure signal (normally caused by the air pump in the programmable system setup) is lower. Accordingly, if a mass flow controller is used the system is more sensitive, i.e. even small defects, such as small velcro cracks, can be detected. The advantage of the programmable system maintaining a constant pressure rate is that this inflation mode is closer to real cuff usage, because when a cuff is used on patient and a measurement is taken during inflation, usually it is inflated at a constant pressure rate rather than constant flow.

Preferably, the pressure-delivery unit of the system and/or the control unit of the device is configured to control the provision of the target pressure in real time.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system detecting a defect in material and/or workmanship of an inflatable cuff according to the present invention.

Fig. 2 shows a schematic diagram of an embodiment of a device for detecting a defect in material and/or workmanship of an inflatable cuff according to the present invention.

Fig. 3A shows a standard inflatable cuff comprising a closing element in an open state.

Fig. 3B shows the standard inflatable cuff of Fig. 3A in a closed state.

Fig. 4 shows an embodiment of a phantom arm device according to the present invention.

Fig. 5 shows a schematic diagram of an embodiment of a method for detecting a defect in material and/or workmanship of an inflatable cuff according to the present invention.

Fig. 6 shows a diagram illustrating the cuff pressure signal.

Fig. 7A shows a raw cuff pressure signal and derived signals.

Fig. 7B shows the cuff pressure signal of Fig. 7A after trend removal.

Fig. 7C shows defects detected in the signals of Fig. 7B, corresponding pressure amplitudes and corresponding pressure within the cuff.

Figs 8A, 8B and 8C show similar signals as Figs. 7A, 7B and 7C, respectively.

Fig. 9A shows average pressure drops for various commercial cuffs during an inflation process.

Fig. 9B shows the root mean square error of the pressure signals of the cuffs shown in Fig. 9A.

Figs. 10A and 10B show average pressure drops and corresponding errors for further commercial cuffs.

DETAILED DESCRIPTION OF THE INVENTION

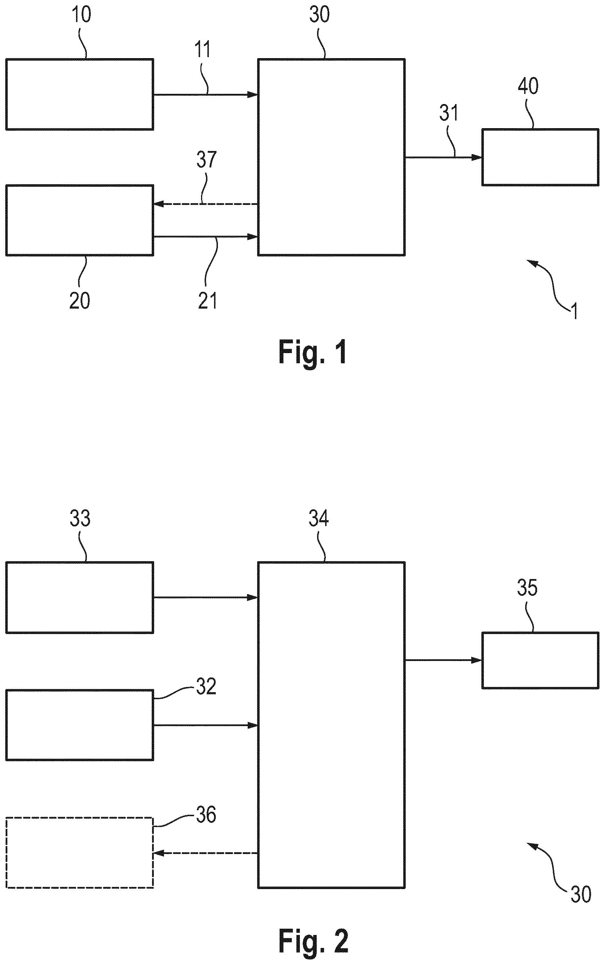

Fig. 1 shows a schematic diagram of an embodiment of a system 1 for detecting a defect in material and/or workmanship of an inflatable cuff according to the present invention.

The system 1 comprises a sensor 10, a pressure delivery unit 20 and a device 30 for detecting a defect in material and/or workmanship of an inflatable cuff. Optionally, the system 1 further comprises a rigid cylinder or a phantom arm device (not shown) and an output interface (40).

In this embodiment a cuff is wrapped around a rigid cylinder and fastened by its closing element. However, the cuff may likewise be wrapped around a stick not having (perfect) cylindrical shape or around a phantom arm device. Furthermore, there is a possibility that the cuff is simply closed without wrapping it around any kind of object.

Within the cuff there is arranged the sensor 10 which is configured to measure the pressure (of gas) within the cuff. The sensor 10 is further configured to provide a cuff pressure signal 11 indicating the pressure measured within the cuff.

A pressure inside the cuff 50 is provided by the pressure-delivery unit 20, i.e. the pressure-delivery unit 20 is configured to inflate or deflate the cuff (via tubes, for example), or to hold a constant pressure within the cuff 50. In this embodiment, the pressure-delivery unit 20 comprises a mass flow controller which is configured to ensure that there is provided a constant flow of gas to the cuff for inflation. Based on the gas flow provided by the pressure-delivery unit 20 and entering the cuff, the pressure-delivery unit 20 calculates a pressure within the cuff and provides a target pressure signal 21 indicating the target pressure in the cuff due to the inflation, wherein the target pressure is the nominal pressure or reference pressure.

The device 30 is configured to obtain, i.e. retrieve or receive, the target pressure signal 21 from the pressure-delivery unit 20 and the cuff pressure signal 11 from the sensor 10. Both signals are then used to detect a defect in the material(s) of the cuff and/or a defect in the workmanship of the cuff (e.g. of the closing element of the cuff). In particular, the device 30 may be configured to compare the pressure signal 11 with target pressure signal 21 and to detect a difference between said signals. If the difference is higher than a predetermined threshold, there is generated a warning signal 31, by the device 30, indicating a defect in the material and/or workmanship of the cuff.

However, the device 30 may likewise be configured to analyze if the target pressure signal indicates a constant rate of inflation of the cuff and to analyze whether the cuff pressure signal comprises discontinuities, particularly changes being higher than a predetermined threshold, and to detect a malfunction if a discontinuity in the signal is detected.

Since the cuff is not attached to a human arm but to a lifeless object, corruptions of the pressure measurement due to movements of the arm, muscle movement or blood pressure oscillations in the arm or any other kind of disturbance from human factors can be avoided. Assuming that the measurement setup is not corrupted, i.e. that the pressure delivery unit 20 and the other parts of the system 1 work properly, artefacts in the cuff pressure signal, particularly a difference between the pressure as measured and the target pressure, may be explained by a deficiency of the cuff 50. Further assuming that the cuff 50 does not have any leakage, there may be a malfunction of the closing element. This is particularly true, if there is a sudden drop or rise in the cuff pressure signal 21.

Optionally, the device 30 of the system 1 may further be configured to control the pressure-delivery unit 20 via a control signal 37, particularly to control inflation and/or deflation of the cuff, for example, the pressure rate at which the cuff is inflated.

The system 1 may further comprise an output interface 40 which may generally be any means that outputs the detected malfunction based on the warning signal provided by the device. The output may be in visual or audible form, e.g. in text form, as image or diagram, as sound or spoken words, etc. For instance, the output interface may be a display, a loudspeaker, a touchscreen, a computer monitor, the screen of a smartphone or tablet, etc.

Fig. 2 shows a schematic diagram of an embodiment of a device 30 for detecting a defect in material and/or workmanship of an inflatable cuff according to the present invention.

The device 30 comprises a target pressure input 32 configured to obtain the target pressure signal 31 and a sensor input 33 configured to obtain the cuff pressure signal 11. The target pressure input 32 and the sensor input 33 may be directly coupled or connected to the pressure-deliver unit 20 and the sensor 10, respectively, or may obtain (i.e. retrieve or receive) the signals from a storage, buffer, network, or bus, etc. The inputs 32 and 33 may thus e.g. be (wired or wireless) communication interfaces or data interfaces, such as a Bluetooth interface, WiFi interface, LAN interface, HDMI interface, direct cable connect, or any other suitable interface allowing signal transfer to the device 30.

The device 30 further comprises a processing unit 34 configured to analyze the cuff pressure signal 11 based on a comparison of the cuff pressure signal 11 with the target pressure signal 21. The processing unit 34 may be any kind of means configured to process and analyze the signals and to detect a malfunction of the closing element of the cuff therefrom. It may be implemented in software and/or hardware, e.g. as a programmed processor or computer or app on a user device such as a smartphone, smartwatch, tablet, laptop, PC, workstation, etc.

The device 30 further comprises an output 35 configured to output the warning signal 31, i.e. a signal indicating that a malfunction of the closing element 51 has been detected. The output 35 may generally be any interface that provides the warning signal 31 e.g. transmits it to another device or provides it for retrieval by another device (e.g. a smartphone, computer, tablet, etc.). It may thus generally be any (wired or wireless) communication or data interface.

Optionally, the device 30 further comprises a control unit 36 which is configured to provide a control signal 37 to a pressure-delivery unit 20 and thus to control the pressure-delivery unit 20 to provide the target pressure within the cuff.

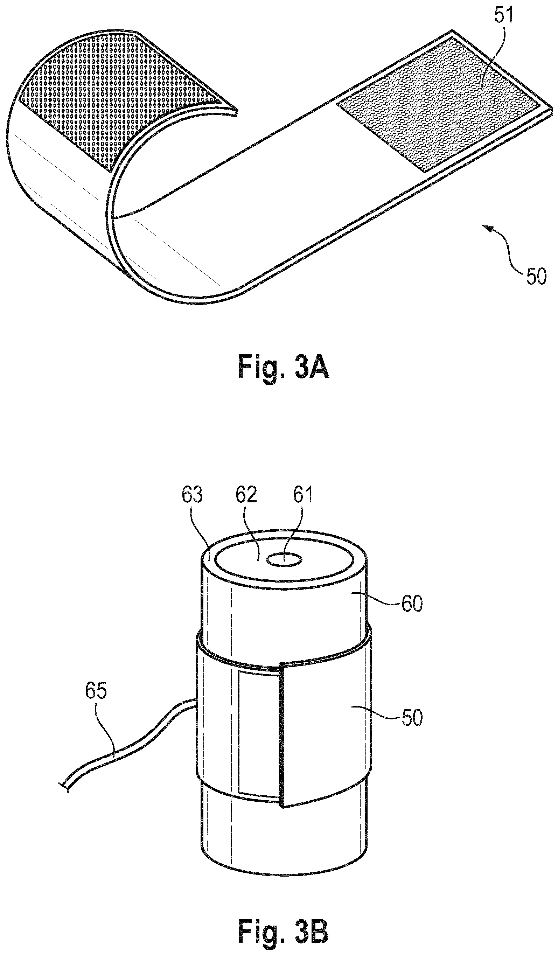

Fig. 3A shows a standard inflatable cuff 50 comprising a closing element 51 in an open state. The quality of the cuff 50 can be assessed using the device 30, system 1 and method 100 according to the present invention. In particular, a defect in the material and/or workmanship of the cuff may be detected. The cuff 50 shown in Fig. 3A comprises a hook and loop fastener as closing element 51. By attaching the hooks to the loops the cuff 50 is closed.

Fig. 3B shows the standard inflatable cuff 50 of Fig. 3A in a closed state. The cuff 50 is wrapped around a phantom arm device 60. The cuff 50 may comprise a tube 65 and a bladder of the cuff (not shown) may be inflated or deflated via said tube by a pressure-delivery unit (not shown).

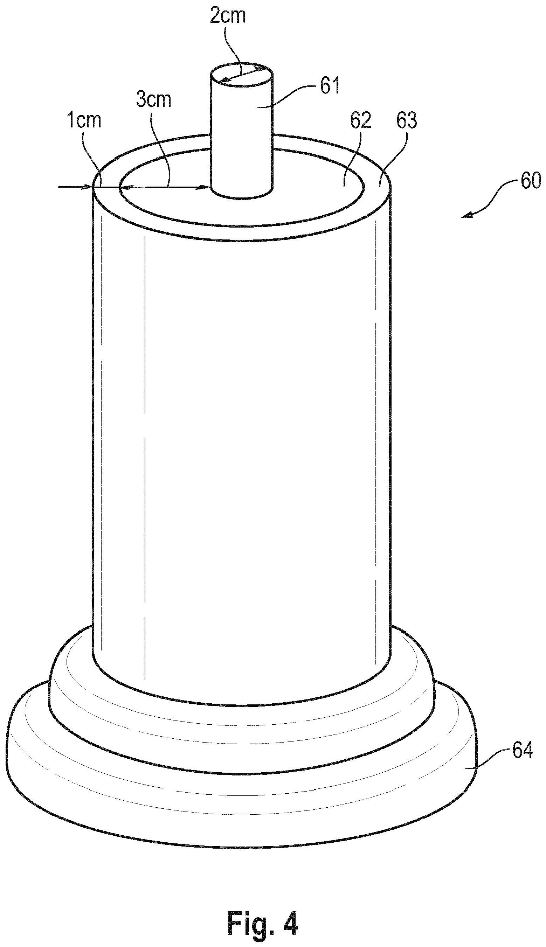

Fig. 4 shows an embodiment of a phantom arm device according to the present invention. The phantom arm device 60 has the form of a cylinder. In this embodiment the diameter of the cylinder is (about) 10cm. However, the diameter of the cylinder form of the phantom arm device 60 may generally range between 7cm and 20cm. The phantom arm device 60 is configured to imitate an arm of a human being and comprises three different materials. A first material is used for an inner cylinder 61 of the phantom arm device 60. The inner cylinder is configured to imitate a bone of a human arm. Therefore, the first material is chosen to be quite hard with a hardness being comparable to the hardness of a human bone. Preferably, polytetrafluoroethylene is used as the first material. Around the shell surface of the inner cylinder 61 there is wrapped a middle layer 62 comprising a second material. The second material is softer than the first material and is configured to imitate the muscles of a human arm. Around the middle layer 62 there is wrapped an outer layer 63 comprising a third material. The material of the outer layer is softer than the second material and is configured to imitate the skin and fat layers of a human being. The middle layer 62 and the outer layer 63 may comprise silicone rubber.

In this embodiment, the inner cylinder has a diameter of 2cm, the thickness of the middle layer is 3cm, and the thickness of the outer layer is 1cm. The phantom arm device 60 further comprises a pedestal 64. Accordingly, the phantom arm device 60 can stand stable.

The length of the middle layer 62 and the outer layer 63 along the axis of the cylinder may be the same or different. For example, the phantom arm device 60 shown in Fig. 4 comprises a middle layer 62 and an outer layer 63 of the same length and an inner cylinder 61 having a longer length than the middle layer and the outer layer.

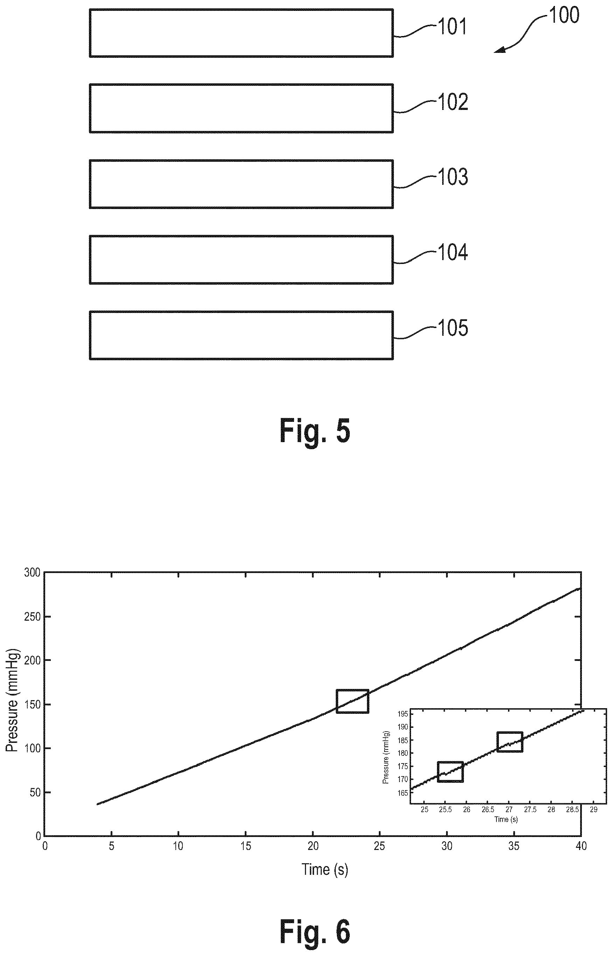

Fig. 5 shows a schematic diagram of an embodiment of a method 100 for detecting a defect in material and/or workmanship of an inflatable cuff according to the present invention. The steps of the method 100 may be carried out by the device 30, wherein the main steps of the method 100 are carried out by the processing unit 34. The method 100 may be implemented as computer program running on a computer or processor.

In a first step 101 the target pressure signal 21 is obtained, e.g. retrieved or received, from the pressure delivery unit 20. In a second step 102, carried out before, after or in parallel to step 101, the cuff pressure signal 11 is obtained, e.g. retrieved or received, from the sensor 10. Subsequently, in step 103 the cuff pressure signal 11 and the target pressure signal 21 are analyzed. Step 103 may particularly comprise comparing both signals. Based on the analysis in step 103, particularly a comparison of both signals, there is detected (determined) a defect in material and/or workmanship of the cuff 50 in step 104. Finally, in step 105, based on a determination of a defect a warning signal 31 is output.

In a preferred embodiment of the method 100 step 103 comprises comparing the cuff pressure signal 11 to the target pressure signal 21, determining if there is a difference between both signals and detecting a defect if the difference is higher than a predetermined threshold.

Fig. 6 shows a diagram illustrating the cuff pressure signal as measured by the sensor 10 in the cuff 50. In particular, Fig. 6 shows the raw (oscillation) pressure within the cuff 50. While the cuff pressure rises constantly over time there can also be seen several drops in the signal. A zoom on the cuff pressure signal 11 is illustrated in the box on the right of Fig. 6. The zoom shows two pressure drops at time points corresponding to (approximately) 25.5s and 27s. Given that the target pressure in the cuff rises constantly (due to constant inflation by a pressure delivery unit, for example), in particularly without any drop, the drops shown in the diagram of Fig. 6 can be regarded as drops caused by defects of the cuff, such as a sudden opening of the closing element allowing the cuff to expand in further directions. In this case, the volume V of air within the cuff may increase, leading to

$$P \propto \frac{1}{V}$$

a drop in the pressure P within the cuff: .

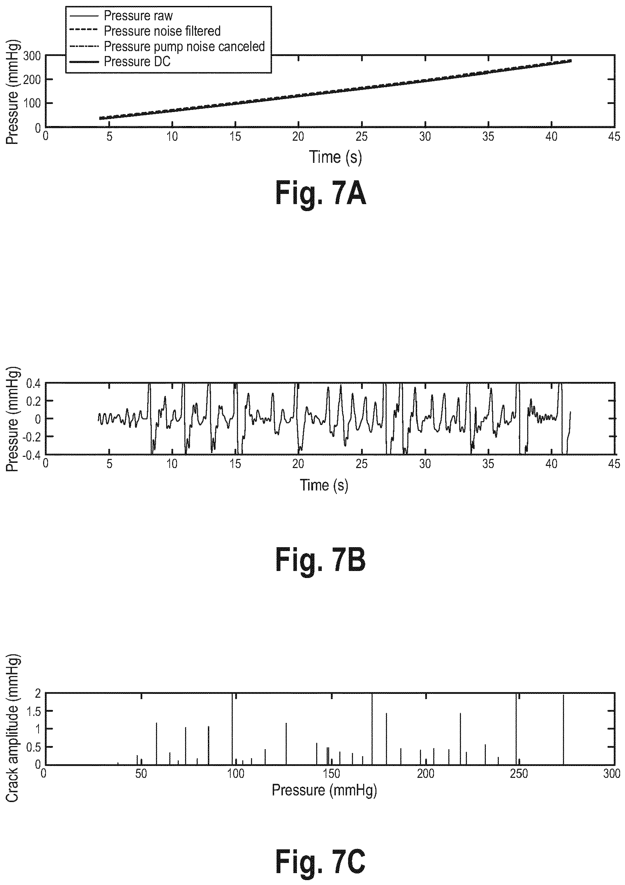

Fig. 7A shows a (raw) cuff pressure signal and derived signals. In particular, Fig. 7A shows a raw cuff pressure signal as measured by a sensor and derived cuff pressure signals (after preprocessing and filtering). The signals are shown for a measurement period of about 45 seconds.

Fig. 7B shows the cuff pressure signal of Fig. 7A after trend removal. In particular, Fig. 7B shows the band-pass-filtered cuff pressure signal. There are revealed (fast) pressure changes in the cuff other than the inflation ramp. The signal shown in Fig. 7B may be used to evaluate the variability in the signal, wherein a higher variability indicates a higher number of velcro cracks or other defects of the cuff.

Fig. 7C shows the pressure levels and amplitudes of detected defects/malfunctions. In particular, the amplitudes may indicate malfunctions of the closing element of the cuff, for example. However, the defects may also be caused by other elements of the cuff. The severity of the defects/malfunctions is indicated by the height of the amplitudes. The higher an amplitude, the stronger the severity of the corresponding malfunction. As can be seen, the cuff used suffers from defects at different levels of pressure within the cuff. The information was evaluated using a threshold as described above (opposed to signal variability and other methods). The amplitude of a defect ("crack") is the difference between the point of highest positive or negative deflection during the crack and the pressure level right before the crack.

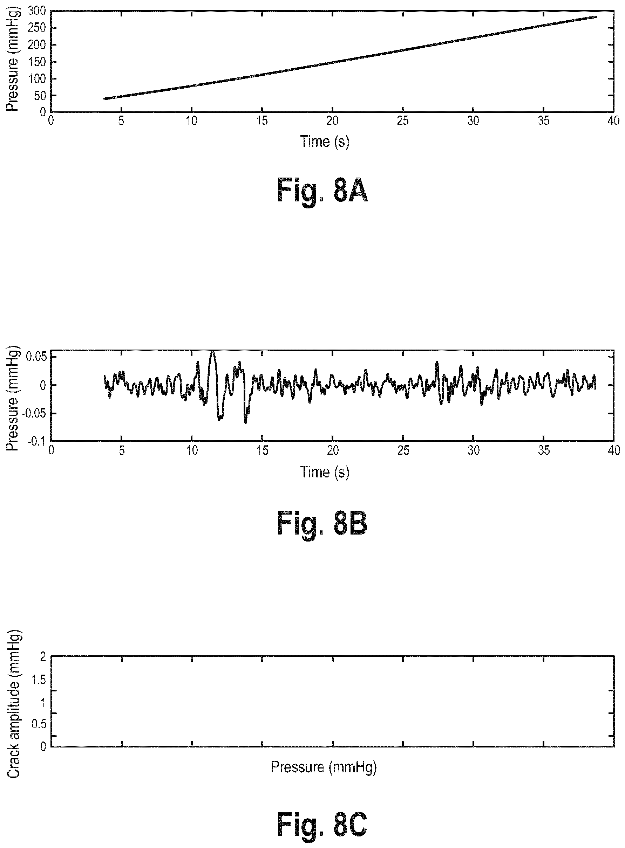

Figs 8A, 8B and 8C show similar signals as Figs. 7A, 7B and 7C, respectively. As can be seen from Fig. 8C the cuff used for the measurements in this case does not reveal any defects.

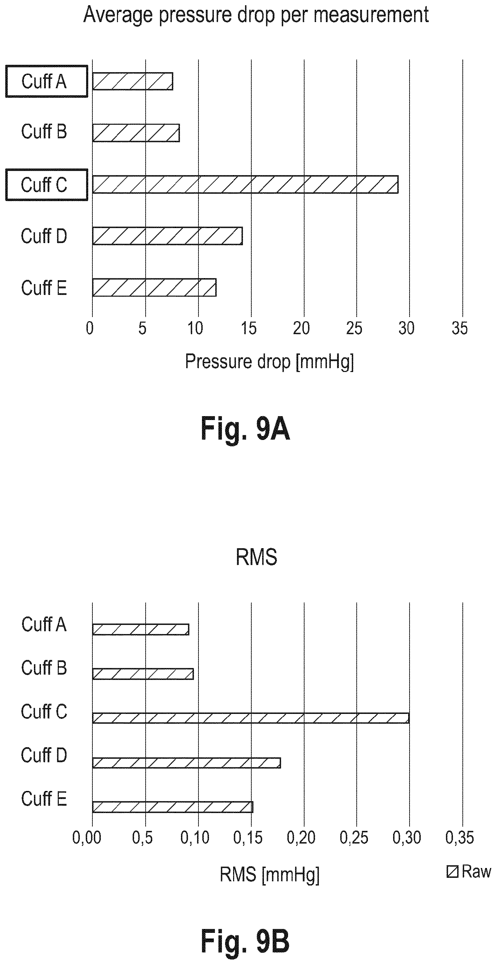

Fig. 9A shows average pressure drops for various commercial cuffs during an inflation process. As can be seen, cuff C suffers from a pressure drop in the cuff pressure signal which is higher than 25 mmHg and caused by a defect of the closing element (for example) during an inflation process.

Fig. 9B shows the root mean square error of the pressure signals of the cuffs shown in Fig. 9A. The RMS (similar to variance or standard deviation, which could be used as well) is a measure of signal variability (unrest). Major source of the variability are the defects of the cuffs. In general, the root-mean-square level of a vector x is defined as

$$x_{rms} = \sqrt{\frac{1}{N} \sum_{n=1}^{N} |x_n|^2}$$

.

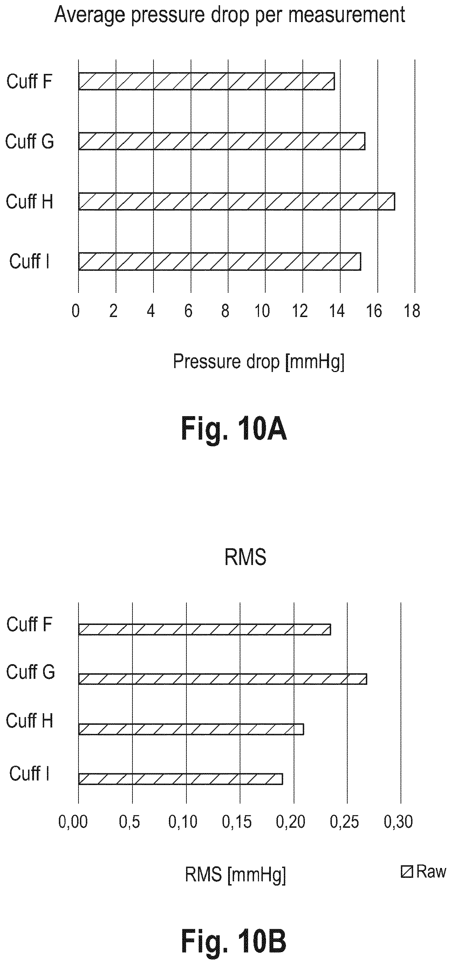

Figs. 10A and 10B show average pressure drops and corresponding errors for further commercial cuffs tested.

As can be seen from Figs. 9A and 10A there was not any cuff tested which did not suffer from a malfunction/defect amounting to a pressure drop less than 5 mmHg during the time of inflation of the cuff.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (30) for detecting a defect in material and/or workmanship of an inflatable cuff (50), the device comprising:

   a target pressure input (32) configured to obtain a target pressure signal (21), the target pressure signal (21) indicating a target pressure within the cuff (50);
   a sensor input (33) configured to obtain a cuff pressure signal (11) from a sensor (10) configured to measure a pressure within the cuff, the cuff pressure signal (11) indicating a pressure within the cuff (50);
   an output (35) configured to output a warning signal (31); and
   a processing unit (34) configured to analyze the cuff pressure signal (11) and the target pressure signal (21), to detect the defect of the cuff (50) based on said analysis, and to control the output (35) to output the warning signal (35) if the defect is detected.

2. Device (30) as claimed in claim 1, further comprising a control unit (36) configured to control a pressure-delivery unit (20) to provide the target pressure within the cuff (50), wherein the control unit (36) is configured to control the pressure-delivery unit (20) to inflate the cuff (50) at a predefined inflation rate and/or to deflate the cuff (50) at a predefined deflation rate and/or to hold the target pressure at a constant level.

3. Device (30) as claimed in claim 1 or 2, wherein the processing unit (34) is configured to detect the defect based on a change in the cuff pressure signal (21), particularly a magnitude and/or shape of the change, more particularly a magnitude and/or shape of a drop or increase in the cuff pressure signal (21).

4. Device (30) as claimed in claim 3, wherein the processing unit (34) is configured to detect the defect if the change in the cuff pressure signal (21) is higher than a threshold, wherein the threshold is a static threshold or an adaptable threshold.

5. Device (30) as claimed in any of the preceding claims, wherein the processing unit (34) is configured to detect the defect based on any of a spectral analysis of the cuff pressure signal (21), a matching of the cuff pressure signal (21) with a template and an evaluation of a variability of a filtered cuff pressure signal.

6. Device (30) as claimed in any of the preceding claims, wherein the processing unit (34) is configured to analyze the cuff pressure signal (21) during any of inflation of the cuff, deflation of the cuff or an inflated state of the cuff.

7. Device (30) as claimed in any of the preceding claims, wherein the cuff (50) is configured to be wrapped around a cylindrical form , the cylindrical form comprising a rigid cylinder or a phantom arm device (50) as claimed in claims 11 and 12.

8. Device (30) as claimed in any of the preceding claims, wherein the defect comprises an opening of a closing element (51), particularly a sudden opening, wherein the closing element (51) comprises any of two surfaces touching each other, a hook and loop fastener, a magnetic closure, and a button.

9. Device (30) as claimed in claims 2 to 8, wherein the processing unit (34) is configured to analyze the cuff pressure signal (21) at different inflation rates and/or at different deflation rates.

10. Device (30) as claimed in any of the preceding claims, wherein the processing unit (34) is configured to count a number of the defects detected.

11. Phantom arm device (60) for detecting a defect in material and/or workmanship of an inflatable cuff (50), the phantom arm device (60) comprising:

    - an inner cylinder (61) comprising a first material;
    - a middle layer (62) comprising a second material, the middle layer being wrapped around a shell surface of the inner cylinder (61); and
- an outer layer (63) comprising a third material, the outer layer (63) being wrapped around the middle layer (62);

wherein a hardness degree of the first material is higher than the hardness degree of the second material; and
wherein the hardness degree of the second material is higher than the hardness degree of the third material.

12. Phantom arm device (60) as claimed in claim 10,

wherein the first material comprises polytetrafluoroethylene and wherein the second and/or third material comprises silicone rubber, wherein a diameter of the inner cylinder (61) is between 1cm and 3 cm, preferably 2 cm, and/or wherein a thickness of the middle layer (62) is between 2 cm and 4 cm, preferably 3 cm, and/or wherein a thickness of the outer layer (63) is between 0.5 mm and 1.5 mm, preferably 1 cm.

13. System (1) for detecting a defect in material and/or workmanship of an inflatable cuff (50), the system (1) comprising:

a sensor (10) configured to measure a pressure within the cuff (50) wrapped around a rigid cylindrical form or a phantom arm device (60);
a pressure-delivery unit (20) configured provide a target pressure within the cuff (50);
a device (30) as claimed in any of claims 1 to 10.

14. Method (100) for detecting a defect in material and/or workmanship of an inflatable cuff, the method comprising:

obtaining a target pressure signal (21), the target pressure signal (21) indicating a target pressure within the cuff (50);
obtaining a cuff pressure signal (11) from a sensor (10) configured to measure a pressure within the cuff (50), the cuff pressure signal (11) indicating a pressure within the cuff (50);
analyzing the cuff pressure signal (11) and the target pressure signal (11);
detecting the defect of the cuff (50) based on said analysis; and
controlling outputting of a warning signal (31) if the defect is detected.

15. Computer program comprising program code means for causing a computer to carry out the steps of the method as claimed in claim 14 when said computer program is carried out on the computer.

**Fig. 1**

**Fig. 2**

Fig. 3A

Fig. 3B

**Fig. 4**

**Fig. 5**

**Fig. 6**

Fig. 7A

Fig. 7B

Fig. 7C

Fig. 8A

Fig. 8B

Fig. 8C

Average pressure drop per measurement

Fig. 9A

RMS

Fig. 9B

## Average pressure drop per measurement

**Fig. 10A**

## RMS

**Fig. 10B**

Europäisches
Patentamt

European
Patent Office

Office européen
des brevets

# EUROPEAN SEARCH REPORT

Application Number

EP 21 20 0851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/211096 A1 (MCEWEN JAMES A [CA] ET AL) 19 August 2010 (2010-08-19) * paragraphs [0036], [0056] * ----- | 1-10, 13-15 | INV. A61B17/135 |
| A | WO 2014/066077 A1 (3M INNOVATIVE PROPERTIES CO [US]; NEUENHAHN MARTIN C [DE] ET AL.) 1 May 2014 (2014-05-01) * page 24, paragraph 2 * ----- | 1 | |

TECHNICAL FIELDS
SEARCHED (IPC)

A61B
A61H

The present search report has been drawn up for all claims

| Place of search | Date of completion of the search | Examiner |
|---|---|---|
| Munich | 16 March 2022 | Angeli, Markus |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

....................................................................
& : member of the same patent family, corresponding
document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-10, 13-15**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
     1. claims: 1-10, 13-15

          Device for detecting a defect in material.
                         ---


     2. claims: 11, 12

          Phantom arm device for simulating standardised behaviour of
          a physical arm.
                         ---
```

## ANNEX TO THE EUROPEAN SEARCH REPORT
## ON EUROPEAN PATENT APPLICATION NO.

EP 21 20 0851

16-03-2022

| Patent document cited in search report | Publication date | Patent family member(s) | Publication date |
|---|---|---|---|
| US 2010211096 A1 | 19-08-2010 | EP 2398403 A1<br>US 2010211096 A1<br>WO 2010094108 A1 | 28-12-2011<br>19-08-2010<br>26-08-2010 |
| WO 2014066077 A1 | 01-05-2014 | CA 2889178 A1<br>CN 104918537 A<br>EP 2911574 A1<br>JP 6316831 B2<br>JP 2016505283 A<br>US 2015297437 A1<br>WO 2014066077 A1 | 01-05-2014<br>16-09-2015<br>02-09-2015<br>25-04-2018<br>25-02-2016<br>22-10-2015<br>01-05-2014 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. HANDLER.** The Importance of Accurate Blood Pressure Measurement. *The Permanente Journal,* 2009, vol. 13 (3), 51-54